(19) **Europäisches Patentamt / European Patent Office / Office européen des brevets**

(11) **EP 2 560 551 B1**

(12) **FASCICULE DE BREVET EUROPÉEN**

(45) Date de publication et mention de la délivrance du brevet:
**03.10.2018 Bulletin 2018/40**

(21) Numéro de dépôt: **11715898.0**

(22) Date de dépôt: **08.04.2011**

(51) Int Cl.:
*A61B 5/00* *(2006.01)*      *A61B 5/11* *(2006.01)*
*A61B 5/103* *(2006.01)*

(86) Numéro de dépôt international:
**PCT/EP2011/055573**

(87) Numéro de publication internationale:
**WO 2011/131497 (27.10.2011 Gazette 2011/43)**

(54) **SYSTEME D'ANALYSE DE FOULEES D'UN UTILISATEUR**

SYSTEM ZUR ANALYSE DER FORTSCHRITTE EINES BENUTZERS

SYSTEM FOR ANALYSING A USER'S STRIDES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.04.2010 FR 1053092**

(43) Date de publication de la demande:
**27.02.2013 Bulletin 2013/09**

(73) Titulaire: **Movea**
**38000 Grenoble (FR)**

(72) Inventeurs:
• **SOUBEYRAT, Cyrille**
**38140 Reaumont (FR)**

• **FRASSATI, Anne**
**38340 Voreppe (FR)**

(74) Mandataire: **Brunelli, Gérald**
**Marks & Clerk France**
**Immeuble Visium**
**22, avenue Aristide Briand**
**94117 Arcueil Cedex (FR)**

(56) Documents cités:
**EP-A1- 1 985 233**      **FR-A1- 2 860 700**
**FR-A1- 2 926 971**      **FR-A1- 2 930 335**
**US-A- 6 122 960**      **US-A1- 2009 079 559**

**Description**

L'invention porte sur un système d'analyse de foulées d'un utilisateur.

De manière générale, l'invention est liée aux domaines de la capture de mouvement, et de la géolocalisation d'individus en déplacement. La capture de mouvement concerne des applications grand public telles des applications de loisir (consoles de jeu interactives, suivi du geste sportif, réalité virtuelle ou réalité augmentée), des applications d'aide à la navigation de piétons (les plus utilisés à l'heure actuelle étant les systèmes de navigation par satellites tel le GPS), des applications d'aide à la mobilité de personnes vulnérables ou temporairement fragilisées par leur environnement (personnes handicapées ou plongées dans l'obscurité), et des applications de fitness (podomètre, calcul de la dépense énergétique ou de la distance parcourue). La capture de mouvement concerne également des applications médicales (suivi des personnes âgées et/ou dépendantes, analyse de la marche pour la rééducation posturale ou aide au diagnostic), des applications de sécurité ou de secours (localisation de pompiers à l'intérieur d'un bâtiment en feu, suivi opérationnel de militaires, ou surveillance de prisonniers), mais aussi des applications à visée commerciale (statistiques sur les trajectoires suivies par les consommateurs dans les centres commerciaux ou les supermarchés, définition d'archétypes d'usage, ou proposition de services commerciaux topo-dépendants).

Il est notamment connu de reconstituer le déplacement d'un objet ou d'une personne munie d'un objet comprenant un émetteur de signaux reconnus par un système d'aide à la navigation par satellites, tel le système GPS, en extérieur, et par un système de radiolocalisation à base de transmissions Ultra Large Bande (ULB) ou WiFi en intérieur.

Dans de nombreuses zones géographiques, externes ou internes, la navigation à base de système d'aide à la navigation par satellites ou de radiolocalisation s'avère néanmoins très délicate, en raison de l'obstruction des signaux radio nécessaires à la mesure de métriques topo-dépendantes, par exemple en cas d'indisponibilité des signaux émis par un ou plusieurs satellites du système d'aide à la navigation, nécessaires à la mesure de pseudo-distances. En pratique, ces situations de blocage ou d'obstruction dégradent fortement la précision de l'information de localisation, et rendent même parfois le service d'aide à la navigation indisponible, comme illustré dans le document "An evaluation of UWB localization under non line-of-sight (NLOS) propagation" (A. Maali, A. Ouldali, H. Mimoun, et G. Baudoin), publié dans Wireless Pervasive Computing, ISWPC, 3rd International Symposium on, pages 379-382, en mai 2008. Généralement, les réalisations citées précédemment présentent un coût, une consommation, un encombrement, et/ou une infrastructure inadaptés, voire rédhibitoires pour des applications grand public.

D'autres systèmes, ont pour but d'élaborer un trajet à partir d'un point de départ donné, en utilisant des capteurs d'attitude (accéléromètre(s), magnétomètre(s), gyromètre) délivrant le plus souvent des mesures inertielles. Ces mesures permettent d'assurer la navigation par des moyens plus ou moins précis et compliqués, tels que décrits, par exemple dans les documents "Inertial head-tracker sensor fusion by a complementary separate-bias kalman filter" (E. Foxlin) publié en mars 1996 dans Virtual Reality Annual International Symposium, Proceedings of the IEEE 1996, pages 185-194; "Detection of spatio-temporal gait parameters by using wearable motion sensors" (K. Kogure L. Seon-Woo, et K. Mase), publié en 2005 dans Engineering in Medicine and Biology Society. IEEE-EMBS 2005. 27th Annual International Conference of the, pages 6836-6839, 2005; "Pedestrian tracking with shoe-mounted inertial sensors" (E. Foxlin), publié en novembre 2005 dans Computer Graphics and Applications, IEEE, 25 :38-46, Nov-Dec 2005; ou "Integration of foot-mounted inertial sensors into a Bayesian location estimation framework" (P. Robertson B. Krach), publié en mars 2008 dans Positioning, Navigation and Communication, 2008. WPNC 2008. 5th Workshop on, pages 55-61. De tels systèmes sont de coût et de complexité élevés, et manquent souvent de précision.

Des consoles de jeu vidéo, telle la Wii, utilisent des capteurs optiques et/ou à ultrasons pour déterminer la trajectoire d'un élément de commande de jeu. Ces systèmes sont couteux et limités.

Il existe également, comme présentés dans les documents "Assessment of walking features from foot inertial sensing" (S. Scapellato F. Cavallo A.M. Sabatini, et C. Martelloni), publié en mars 2005 dans Biomedical Engineering, IEEE Transactions on, pages 486- 494, ou "Multisensor approach to walking distance estimation with foot inertial sensing" (D. Alvarez A.M Lopez J. Rodriguez-Uria J.C. Alvarez, et R.C. Gonzalez), publié en août 2007 dans Engineering in Medicine and Biology Society, 2007, EMBS 2007. 29th Annual International Conference of the IEEE, pages 5719-5722, des systèmes dans lesquels on suppose deux accéléromètres et un gyromètre disposés dans le plan sagittal d'un utilisateur, qui permettent de déterminer l'attitude de ce capteur dans le plan. L'accélération est intégrée dans l'axe du pied. Ces systèmes ont un coût réduit, mais présentent d'autres inconvénients. D'une part, ils supposent que les capteurs sont parfaitement placés dans le plan sagittal, ce qui est quasiment impossible à réaliser concrètement par l'utilisateur, et provoque une erreur d'estimation liée au mauvais positionnement du ou des capteurs. D'autre part, ils supposent que la marche a lieu dans le plan sagittal, ce qui peut ne pas être le cas, lors d'une marche sur le côté, par exemple.

Le document, "An innovative shoe-mounted pedestrian navigation system" (K. Fyfe, Gérard Lachapelle, R. Stirling, et J. Collin), publié en avril 2003 dans Proc. European Navigation Conf. (GNSS), CD-ROM, Austrian Inst. of Navigation, divulgue un système muni de trois accéléromètres et d'un quatrième accéléromètre afin de calculer l'angle subi par le capteur. Cet accéléromètre supplémentaire est placé sur le même élément mobile, mais à distance de ce dernier. Les deux capteurs voient donc la même rotation et le même déplacement. Une telle structure permet d'estimer la vitesse de rotation de l'élément mobile, en rotation autour d'une direction sensiblement constante, en se passant d'un gyromètre. Ce système a un coût réduit du au remplacement d'un gyromètre mono-axe par un couple d'accéléromètres par rapport aux systèmes avec des gyromètres proposés par Alvarez et al et Scapellato et al. Cependant, un tel système présente les mêmes inconvénients que l'exemple cité ci-dessus, car seule la rotation autour de l'axe orthogonal au plan sagittal est prise en compte. En outre, l'estimation de la rotation par un couple d'accéléromètres, tout comme celle réalisée à l'aide d'un gyromètre, présente des dérives importantes. Un cap est également déterminé à l'aide d'un magnétomètre, par exemple lorsque le pied est posé, ce qui donne la direction du pied et non pas la direction du déplacement recherchée.

La demande de brevet français FR 0857181 porte sur un dispositif de détermination d'une caractéristique trajectoire formée de positions successives d'un accéléromètre triaxial lié de manière solidaire à un élément mobile, entre un premier instant d'immobilité ou de quasi-immobilité et un deuxième instant d'immobilité de quasi immobilité de l'accéléromètre triaxial, consécutif audit premier instant, ledit dispositif comprenant, en outre, un capteur additionnel triaxial de mesure d'un vecteur d'un champ vectoriel sensiblement constant entre lesdits premier et deuxième instants d'immobilité, dans un repère global fixe lié au repère terrestre. Le capteur additionnel, par exemple un magnétomètre triaxial, est lié de manière solidaire audit élément mobile et fixe dans le repère de l'accéléromètre, et le dispositif comprend des moyens de commande. Lesdits moyens de commande comprennent :

- des premiers moyens de détermination desdits premier et deuxième instants d'immobilité de l'accéléromètre triaxial ;
- des deuxièmes moyens de détermination d'un axe de rotation sensiblement invariant de l'accéléromètre triaxial entre lesdits premier et deuxième instants d'immobilité, et d'un plan orthogonal audit axe de rotation sensiblement invariant, dans un repère mobile lié à l'accéléromètre ou au capteur additionnel à partir des vecteurs délivrés par l'accéléromètre triaxial, ou à partir des vecteurs délivrés par le capteur additionnel ;
- des premiers moyens de calcul, auxdits instants successifs, de premières projections orthogonales sur ledit plan des vecteurs délivrés par l'accéléromètre triaxial et des vecteurs délivré par le capteur additionnel dans ledit repère mobile ;
- des troisièmes moyens de détermination, auxdits instants successifs, de la rotation de passage dudit repère mobile audit repère global fixe, à partir desdites premières projections orthogonales des vecteurs délivrés par le capteur additionnel ;
- des deuxièmes moyens de calcul, auxdits instants successifs, de deuxièmes projections orthogonales dans ledit repère global fixe, des premières projections orthogonales dans ledit plan des vecteurs délivrés par l'accéléromètre triaxial, fournies par lesdits premiers moyens de calcul ;
- des troisièmes moyens de calcul pour soustraire à chaque deuxième projection orthogonale dans ledit repère global fixe le vecteur moyen sur lesdits instants successifs, de manière à obtenir les accélérations centrées dans ledit plan, dépourvues de l'influence de la gravité terrestre et de dérives dudit dispositif, dans ledit repère global fixe ; et
- des quatrièmes moyens de calcul d'une caractéristique de la trajectoire à partir des accélérations centrées.

Un tel dispositif permet de déterminer une trajectoire planaire d'un accéléromètre triaxial lié à un élément mobile, notamment un pied, pour une foulée, pour ainsi reconstruire une trajectoire d'un ensemble de foulées successives. L'élément mobile peut être une autre partie du corps humain, comme une main, une partie d'un corps animal, ou une partie d'un corps artificiel tel un robot ou une souris informatique.

Un tel dispositif permet d'obtenir des informations globales liées au déplacement de l'utilisateur dans un plan sagittal. Il ne permet pas d'obtenir des informations, en trois dimensions, relatives à la foulée d'un utilisateur, notamment d'un coureur.

Un but de l'invention est de pallier ces problèmes. L'invention est définie par les revendications. Selon un aspect de l'invention, il est proposé un système d'analyse de foulées d'un utilisateur, comprenant :

- un premier ensemble capteur muni d'un boîtier, d'un magnétomètre triaxial lié fixement audit boîtier, et d'un référentiel mobile ;
- des premiers moyens de fixation adaptés pour fixer le premier ensemble capteur à un segment de jambe de l'utilisateur ; et
- des moyens de traitement adaptés pour calculer l'angle de lacet et/ou de tangage et/ou de roulis dudit ensemble capteur, et pour calculer la ou les variations temporelles angulaires correspondantes, dans un référentiel global fixe lié au référentiel terrestre, à partir des mesures dudit magnétomètre triaxial délivrées dans ledit référentiel mobile.

Lesdits moyens de traitement comprennent :

- des premiers moyens de calcul de la variation temporelle de l'angle de tangage du premier ensemble capteur à partir des mesures dudit magnétomètre triaxial délivrées dans ledit référentiel mobile ; et

- des premiers moyens de détermination d'un instant de début d'une foulée et d'un instant de fin de ladite foulée et de phases de ladite foulée, à partir de ladite variation temporelle de l'angle de tangage du premier ensemble capteur fournie par lesdits premiers moyens de calcul.

Un tel système permet d'obtenir des informations, en trois dimensions, relatives à la foulée d'un utilisateur.

En outre, les moyens de traitement peuvent être internes ou externes au boîtier, et lorsqu'ils sont externes, ils peuvent être embarqués à bord d'un téléphone portable ou d'un ordinateur portable.

On entend par jambe, un membre inférieur complet. De manière préférentielle, le segment de jambe auquel peut être fixé le premier ensemble capteur EC1, est le cou-de-pied ou le métatarse du pied, ou le tibia.

Le premier ensemble capteur peut être calibré, si nécessaire, pour que le référentiel mobile soit tel que deux axes forment un plan vertical dans un référentiel global fixe lié au référentiel terrestre.

Dans la suite de la description, le terme référentiel peut être associé à un repère associé au référentiel.

Il est ainsi possible, par exemple, de déterminer avec précision le temps de contact au sol (entre la pose du pied au sol et l'instant où le pied va quitter le sol) ou l'accélération du pied à l'instant instant où le pied va quitter le sol, qui sont d'importantes caractéristiques de la foulée d'un coureur.

Dans un mode de réalisation, ledit premier ensemble capteur est, en outre, muni d'un accéléromètre triaxial lié fixement audit boîtier, et lesdits moyens de traitement comprennent des deuxièmes moyens de détermination de valeurs de paramètres représentatifs d'une phase de ladite foulée ou de la foulée complète, en trois dimensions, dans le référentiel global fixe.

Ainsi la précision du système est améliorée, en obtenant des informations liées à des phases de la foulée.

Selon un mode de réalisation, une foulée comprenant une phase d'appui du pied auquel est fixé le premier ensemble capteur, suivi d'une phase de cycle arrière dans laquelle ledit pied remonte du sol en direction du dos de l'utilisateur, et d'une phase de cycle avant dans laquelle ledit pied se dirige devant l'utilisateur, lesdits deuxièmes moyens de détermination comprenant des premiers moyens d'estimation d'un référentiel lié au premier ensemble capteur, pour la foulée et/ou par phase de la foulée, à partir d'une décomposition en valeurs singulières, de la matrice à trois colonnes formée par des vecteurs délivrés par ledit magnétomètre triaxial à des instants successifs, dans le référentiel mobile lié au premier ensemble capteur.

L'invention permet de déterminer un référentiel variable lié au premier ensemble capteur, pour l'ensemble de la foulée et par phase de la foulée, les phases d'une foulée sont définies plus loin dans la description.

Les deux vecteurs associés aux deux plus grandes valeurs singulières forment le plan principal de la foulée.

Dans un mode de réalisation, lesdits moyens de traitement comprennent des deuxièmes moyens de calcul de projections orthogonales des vecteurs de mesures délivrés par l'accéléromètre triaxial et par le magnétomètre triaxial sur les axes du référentiel lié au premier ensemble capteur sur la foulée.

Ainsi, il est possible d'obtenir l'évolution des axes du magnétomètre vu d'un référentiel réputé fixe durant la foulée, et d'en déduire l'évolution des angles décrivant l'attitude spatiale de l'ensemble capteur, donc du segment de jambe auquel il est fixé, par exemple le pied.

Selon un mode de réalisation, le référentiel global fixe lié au référentiel terrestre est ladite projection orthogonale des vecteurs délivrés par le magnétomètre triaxial dans le référentiel lié au premier ensemble capteur pour la foulée.

En effet, cela est possible car sur une foulée, le champ magnétique reste sensiblement constant dans un référentiel global fixe lié au référentiel terrestre.

Dans un mode de réalisation, lesdits deuxièmes moyens de détermination comprennent des troisièmes moyens de détermination des angles de tangage, de roulis et de lacet du premier ensemble capteur, à partir de ladite projection orthogonale des vecteurs délivrés par le magnétomètre triaxial dans le référentiel lié au premier ensemble capteur pour la foulée , et de détermination des vitesses angulaires correspondantes de tangage, de roulis et de lacet.

Par défaut on détermine ainsi les six degrés de liberté, puis ensuite on teste les quels peuvent être négligés.

Selon un mode de réalisation, lesdits deuxièmes moyens de détermination comprennent des moyens de comparaison desdites vitesses angulaires de tangage, de roulis et de lacet avec un seuil.

On peut ainsi tester quels degrés de liberté peuvent être négligés.

Dans un mode de réalisation, lesdits deuxièmes moyens de détermination comprennent des moyens d'utilisation du référentiel lié au premier ensemble capteur pour la phase en cours, lorsqu'au moins deux desdites valeurs absolues des trois vitesses angulaires sont supérieures audit seuil, pour redresser l'orientation par compensation des déviations de la trajectoire, dues aux rotations, par rapport au référentiel global en utilisant ledit référentiel lié audit premier ensemble capteur pour la phase en cours à la place du référentiel lié au premier ensemble capteur pour la foulée.

Le redressement d'orientation s'entend par déterminer la trajectoire dans un référentiel adéquat, différent du référentiel calculé pour l'ensemble de la foulée, à cause des vitesses angulaires non négligeables qui font dévier la trajectoire du référentiel de la foulée complète.

Ainsi, la trajectoire reconstruite est composée de trajectoires unitaires, pour chaque phase de la foulée, dont les référentiels respectifs sont adaptés au nombre de degrés de liberté à prendre en compte pour chacune des phases.

Selon un mode de réalisation, lesdits premiers moyens de détermination sont adaptés pour identifier des instants caractéristiques de la foulée de l'utilisateur par détection d'extrema locaux de la variation temporelle de l'angle de tangage du premier ensemble capteur.

Il est ainsi aisé de déterminer ces instants caractéristiques de la foulée.

Dans un mode de réalisation, les deuxièmes moyens de détermination comprennent des troisièmes moyens de calcul pour soustraire, au vecteur correspondant à la projection orthogonale dans le référentiel global du vecteur de mesures de l'accéléromètre triaxial délivré par les deuxièmes moyens de calcul, le vecteur moyen sur la foulée de la projection orthogonale dans le référentiel global du vecteur de mesures de l'accéléromètre triaxial délivré par les deuxièmes moyens de calcul, de manière à obtenir le vecteur centré des accélérations dans le référentiel global.

L'obtention des accélérations centrées dans le référentiel global, permet de s'abstraire de l'influence de la gravité.

Selon un mode de réalisation, les deuxièmes moyens de détermination comprennent des quatrièmes moyens de calcul d'une double intégration temporelle dudit vecteur centré des accélérations dans le référentiel global délivré par lesdits troisièmes moyens de calcul, de manière à obtenir la trajectoire du premier ensemble capteur dans ledit référentiel global, sur la foulée.

Ainsi, on obtient une caractéristique en trois dimensions de la foulée de l'utilisateur, pour chaque phase de la foulée.

En outre, les deuxièmes moyens de détermination comprennent des moyens de correction des dérives, en supposant que le pied de l'utilisateur a une orientation identique en début et fin de foulée, par redressement, de manière à avoir des coordonnées verticales et transversales identiques en début et fin de foulée.

Ainsi, la trajectoire est redressée, sans avoir recours à des calculs d'angles entre le référentiel global et le référentiel terrestre.

Le système peut également comprendre un deuxième ensemble capteur semblable au premier ensemble capteur, et des deuxièmes moyens de fixation adaptés pour fixer le deuxième ensemble capteur à un segment de l'autre jambe de l'utilisateur.

Le système peut alors, en outre, comprendre des moyens de synchronisation pour comparer les mesures effectuées par le ou lesdits ensembles capteurs d'une jambe par rapport à l'autre.

Ainsi, il est d'une part possible de caractériser la fin de cycle avant d'un pied en mesurant la contribution de l'accélération à l'instant où l'autre pied va quitter le sol.

D'autre part, la mesure synchrone des deux trajectoires des ensembles capteurs liés aux différentes jambes de l'utilisateur permet de déterminer la présence d'asymétries temporelles et spatiales de la foulée du coureur.

En outre, le système peut comprendre un troisième ensemble capteur similaire au premier ensemble capteur, et des troisièmes moyens de fixation adaptés pour fixer le troisième ensemble capteur à mi-hauteur de l'utilisateur.

Ainsi, une mesure des trajectoires des deux segments de jambe, notamment des deux pieds, effectuée de manière synchrone avec la mesure de l'oscillation du centre de masse du coureur dans le plan sagittal permet de déterminer la phase de course à modifier de manière à diminuer l'amplitude de ladite oscillation.

Selon un autre aspect de l'invention, il est également proposé un procédé d'analyse de foulées d'un utilisateur, dans lequel :

- on calcule la variation temporaire de l'angle de tangage d'un premier ensemble capteur, comprenant un magnétomètre triaxial, fixé à un segment de jambe de l'utilisateur, à partir des mesures dudit magnétomètre triaxial ;
- on détermine un instant de début d'une foulée et un instant de fin de ladite foulée et des phases de ladite foulée, à partir de calcul de la variation temporelle de l'angle de tangage du premier ensemble capteur ; et
- on détermine des valeurs de paramètres représentatifs d'une phase de ladite foulée, en trois dimensions, dans un référentiel global fixe lié au référentiel terrestre.

L'invention sera mieux comprise à l'étude de quelques modes de réalisation décrits à titre d'exemples nullement limitatifs et illustrés par les dessins annexés sur lesquels :

- les figures 1a, 1b, et 1c illustrent schématiquement la décomposition d'une foulée, selon un aspect de l'invention ;
- la figure 2 illustre schématiquement le déplacement d'un ensemble capteur fixé à un pied d'un utilisateur, selon un aspect de l'invention ;
- les figures 3a et 3b illustrent schématiquement un système selon un aspect de l'invention ;
- la figure 4 illustre plus en détails un module de traitement selon un aspect de l'invention ; et
- les figures 5a, 5b, 5c, 5d et 5e illustrent des éléments du module de traitement de la figure 4.

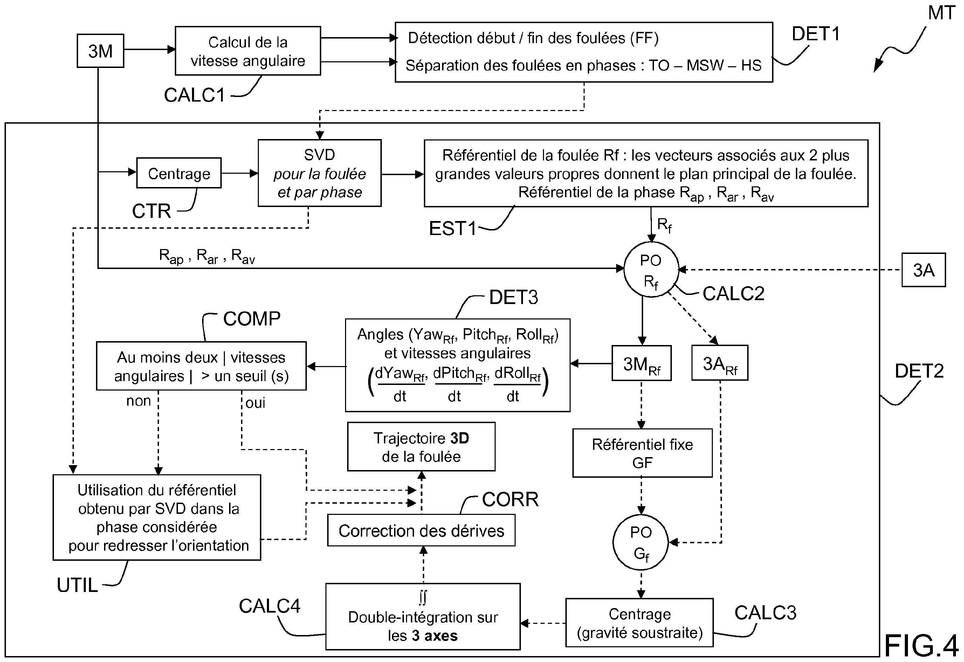

Dans l'ensemble de figures, les éléments ayants les mêmes références sont similaires. Dans la présente description, on décrit un système complexe, notamment illustré par la figure 4, mais l'invention couvre également une réalisation moins complexe comprenant uniquement les éléments permettant de calculer l'angle de lacet et/ou de tangage et/ou de roulis, ainsi que la ou les variations temporelles angulaires correspondantes, dans un référentiel global fixe lié au référentiel terrestre, à partir des mesures d'un magnétomètre triaxial délivrées dans un référentiel lié au magnétomètre. En effet, toute la partie du système illustré sur la figure 4, dont les flèches entre éléments sont représentées en pointillés, est optionnelle, pour obtenir le système complexe, le reste de la figure représente un mode de réalisation moins complexe permettant de déterminer l'angle de lacet (Yaw$_{GF}$) et/ou de tangage (Pitch$_{GF}$) et/ou de roulis (Roll$_{GF}$) dudit premier ensemble capteur (EC1), et pour calculer la ou les variations temporelles angulaires correspondantes

$$(\frac{d\,Yaw_{GF}}{dt}, \frac{d\,Pitch_{GF}}{dt}, \frac{d\,Roll_{GF}}{dt}),$$

dans un référentiel global fixe lié au référentiel terrestre, à partir des mesures dudit magnétomètre triaxial délivrées dans ledit référentiel mobile.

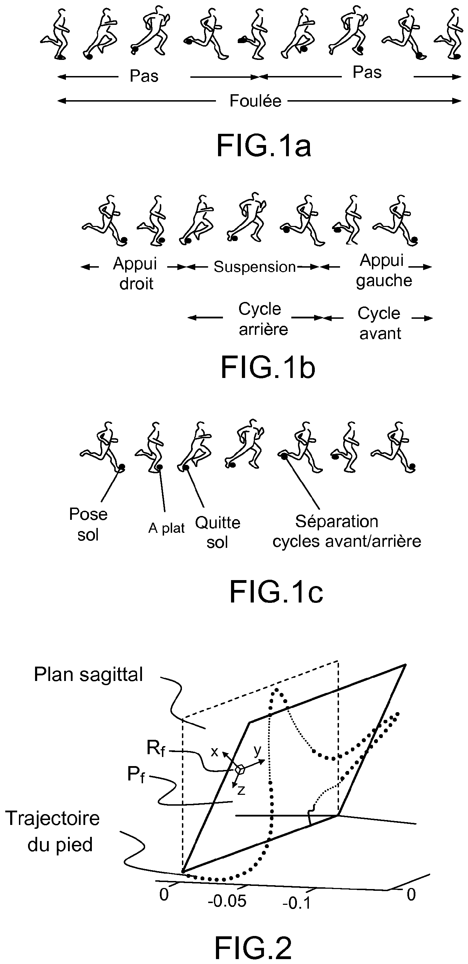

Tel qu'illustré sur la figure 1a, une foulée d'un coureur est décomposée en deux pas successifs (pose du pied gauche et du pied droit). Une foulée est une phase de course qui se déroule entre deux contacts au sol successifs d'un même pied.

Sur la figure 1b, sont représentées les phases d'une foulée. La foulée se décompose en deux phases distinctes. La phase d'appui pendant laquelle un des deux pieds est en contact avec le sol, et La phase de suspension pendant laquelle les deux pieds sont en l'air. Pendant une phase de vol d'un pied (suspension et appui de l'autre pied) se divise elle-même en deux phases : une phase de cycle arrière durant laquelle le pied remonte du sol en direction du dos du coureur, et une phase de cycle avant durant laquelle le pied se dirige dans le sens de la course, ou devant l'utilisateur.

La figure 1c représente les événements ou instants caractéristiques d'une foulée considérés. Au cours de la foulée, la trajectoire du pied est caractérisée par quatre instants caractéristiques notables :

- maintien du pied à plat ou FF pour "Foot Flat" en langue anglaise correspondant au maintien du bassin au dessus du pied ;
- instant où le pied va quitter le sol ou TO pour "Toe Off" en langue anglaise ;
- instant auquel le pied repart vers l'avant ou MSW pour "Mid Swing" durant la phase de suspension ; et
- pose du pied au sol ou HS pour "Heel Strike" en langue anglaise correspondant au début de la phase d'amortissement qui s'achève lorsque le bassin passe au dessus du pied.

La figure 2 illustre le plan principal $P_f$ et le référentiel $R_f$ de la foulée. Au cours de la foulée, la trajectoire du pied se déroule au voisinage d'un plan $P_f$ étant le plan principal de la foulée. Ce plan présente un angle en général non nul vis-à-vis du plan sagittal qui est défini par la direction de la foulée au sol et la verticale. Le référentiel de la foulée est construit à partir de deux axes du plan principal $P_f$ de la foulée et d'un axe normal.

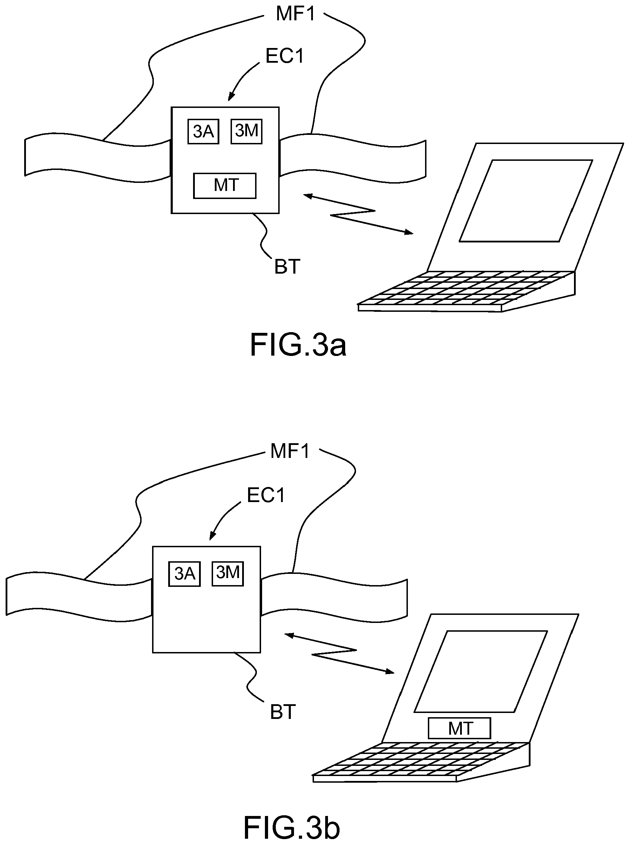

La figure 3a illustre un système d'analyse de foulées d'un utilisateur, comprenant un premier ensemble capteur EC1 muni d'un boîtier BT, d'un magnétomètre triaxial 3M lié fixement au boîtier BT. L'ensemble capteur EC1 est muni d'un référentiel mobile LF, tel que deux axes (y, z) forment un plan vertical dans un référentiel global fixe GF lié au référentiel terrestre. Le système comprend également des premiers moyens de fixation MF1, par exemple des éléments élastiques munis de scratchs, adaptés pour fixer le premier ensemble capteur EC1 à un segment d'une jambe de l'utilisateur, par exemple un pied de l'utilisateur. En outre, le premier ensemble capteur EC1 comprend un accéléromètre triaxial 3A optionnel lié fixement au boîtier BT. En effet, dans le système décrit, outre l'extraction des angles et vitesses angulaires, on détermine la trajectoire en trois dimensions du premier ensemble capteur EC1, et donc du segment de jambe auquel il est lié fixement.

Le système comprend, en outre, un module de traitement MT.

En l'espèce, le module de traitement MT est interne au boîtier BT, et peut transmettre des résultats à un terminal extérieur, tel un ordinateur ou un téléphone portable, pour les afficher. En variante, un élément d'affichage des résultats pourrait être directement intégré au boîtier BT.

En variante, tel qu'illustré sur la figure 3b, le module de traitement MT peut être déporté dans un terminal extérieur, tel un ordinateur ou un téléphone portable.

La figure 4 illustre plus en détails un mode de réalisation du module de traitement MT.

Un premier module de détermination DET1 permet de déterminer un instant de début d'une foulée, un instant de fin de ladite foulée, et des phases de ladite foulée, à partir d'une variation temporelle de l'angle de tangage Pitch du premier ensemble capteur EC1. Un deuxième module de détermination DET2 permet de déterminer des valeurs de paramètres représentatifs d'une phase de ladite foulée, ou de la foulée complète, en trois dimensions, dans le référentiel global fixe GF.

Le module de traitement MT comprend un premier module de calcul CALC1 de la variation temporelle de l'angle de tangage Pitch du premier ensemble capteur EC1 dans le référentiel mobile LF à partir des mesures du magnétomètre triaxial 3M.

Le deuxième module de détermination DET2 comprend un premier module d'estimation EST1 d'un référentiel lié au premier ensemble capteur EC1, pour la foulée $R_f$ et par phase $R_{ap}$, $R_{ar}$, $R_{av}$ de la foulée, à partir d'une décomposition en valeurs singulières ou SVD pour "singular value decomposition" en langue anglaise, dans ledit référentiel mobile LF lié au premier ensemble capteur EC1, de la matrice à trois colonnes formée par des vecteurs délivrés par le magnétomètre triaxial 3M à des instants successifs dans le référentiel mobile LF.

Le module de traitement MT comprend un deuxième module de calcul CALC2 de projections orthogonales PO des vecteurs de mesures délivrés par l'accéléromètre triaxial 3A et par le magnétomètre triaxial 3M sur les axes du référentiel lié au premier ensemble capteur sur la foulée $R_f$.

Le référentiel global fixe GF, pour "global frame" en langue anglaise, lié au référentiel terrestre, est la projection orthogonale PO des vecteurs délivrés par le magnétomètre triaxial 3M dans le référentiel lié au premier ensemble capteur EC1 pour la foulée $R_f$.

Le deuxième module de détermination DET2 comprend un troisième module de détermination DET3 des angles de tangage $Pitch_{R_f}$ de roulis $Roll_{R_f}$ et de lacet $Yaw_{R_f}$ du premier ensemble capteur EC1, dans le référentiel $R_f$, à partir de la projection orthogonale PO des vecteurs délivrés par le magnétomètre triaxial 3M dans le référentiel LF lié au premier ensemble capteur EC1 pour la foulée , et de détermination des vitesses angulaires correspondantes de tangage

$$\frac{d\,Pitch_{R_f}}{dt}, \text{ de roulis } \frac{d\,Roll_{R_f}}{dt} \text{ et de lacet } \frac{d\,Yaw_{R_f}}{dt}.$$

Le deuxième module de détermination DET2 comprennent des moyens de comparaison COMP des vitesses angulaires de tangage

$$\frac{d\,Pitch_{R_f}}{dt}, \text{ de roulis } \frac{d\,Roll_{R_f}}{dt} \text{ et de lacet } \frac{d\,Yaw_{R_f}}{dt} \text{ avec un seuil S.}$$

Le deuxième module de détermination DET2 comprend un module d'utilisation UTIL du référentiel $R_{ap}$, $R_{ar}$, $R_{av}$ lié au premier ensemble capteur EC1 pour la phase en cours, lorsqu'au moins deux desdites valeurs absolues des trois vitesses angulaires sont supérieures audit seuil S, pour redresser l'orientation, c'est-à-dire pour replacer la trajectoire durant la phase en cours de la foulée, en utilisant le référentiel $R_{ap}$, $R_{ar}$, ou $R_{av}$ lié à l'ensemble capteur EC1 pour la phase en cours à la place du référentiel $R_f$ lié au premier ensemble capteur EC1 pour l'ensemble de la foulée.

Le premier module de détermination DET1 est adapté pour identifier des instants caractéristiques HS, FF, TO, MSW de la foulée de l'utilisateur par détection d'extrema locaux de la variation temporelle $\dfrac{d\,Pitch}{dt}$ de l'angle de tangage Pitch dans le référentiel lié au premier ensemble capteur EC1.

En outre, la séparation en phases par le premier ensemble capteur EC1 permet de distinguer les moments de la foulée pendant lesquels la trajectoire en trois dimensions peut être théoriquement estimée, des moments où le nombre de degrés de liberté trop important l'en empêche (ces nombres sont obtenus par extraction des vitesses angulaires).

La phase d'appui va de la pose du pied au sol HS à l'instant où le pied va quitter le sol TO : le nombre de degrés de liberté majoritaires est deux, car le roulis Roll et le tangage Pitch, les accélérations et le lacet Yaw y sont négligeables.

La phase de cycle arrière va de l'instant ou le pied va quitter le sol TO à l'instant séparant le cycle arrière du cycle avant MSW : le nombre de degrés de liberté est généralement de six, car les trois accélérations et les trois angles ne sont pas négligeables.

La phase de cycle avant va de l'instant séparant le cycle arrière du cycle avant MSW à la pose du pied au sol HS : le roulis Roll et le tangage Yaw y sont négligeables, donc il est quatre degrés de liberté majoritaires.

L'avantage de la connaissance de ce nombre de degrés de liberté est de pouvoir valider ou non la trajectoire reconstruite obtenue. Dans les phases d'appui et de cycle avant, le fait d'avoir moins de cinq degrés de liberté permet de reconstruire la trajectoire en trois dimensions. Dans la phase de cycle arrière, le nombre de degrés de liberté étant généralement de six, une estimation plus approfondie des paramètres doit être mise en oeuvre afin d'avoir la meilleure estimation possible de la trajectoire en trois dimensions au cours de cette phase.

Le découpage de la foulée permet de traiter séparément les signaux, on peut calculer un référentiel de la foulée $R_f$ différent pour chacune des phases. Il y aura ainsi trois décompositions en valeurs singulières SVD effectuées au lieu d'une, et les projections sont faites phase par phase. En effet, dans la phase d'appui au sol, par exemple, le référentiel de la foulée $R_f$ peut être très différent du référentiel global GF estimé sur une foulée entière. Le fait de changer de référentiel permet de donner plus de précisions dans l'orientation du mouvement.

Le deuxième module de détermination DET2 comprend, un troisième module de calcul CALC3 pour soustraire, au vecteur correspondant à la projection orthogonale PO dans le référentiel global GF du vecteur de mesures de l'accéléromètre triaxial 3A délivré par le deuxième module de calcul CALC2, le vecteur moyen sur la foulée de la projection orthogonale PO dans le référentiel global GF du vecteur de mesures de l'accéléromètre triaxial 3A délivré par le deuxième module de calcul CALC2, de manière à obtenir le vecteur centré des accélérations dans le référentiel global GF.

Ce centrage permet d'obtenir des accélérations centrées, i.e. dépourvues de l'influence de la gravité terrestre dans ledit repère global fixe.

Le deuxième module de détermination DET2 comprend des quatrièmes moyens de calcul CALC4 d'une double intégration temporelle dudit vecteur centré des accélérations dans le référentiel global GF délivré par le troisième module de calcul CALC3, de manière à obtenir la trajectoire du premier ensemble capteur EC1 dans ledit référentiel global GF, sur la foulée.

En outre, un module de correction CORR des dérives en supposant que le pied a une orientation identique en début et fin de foulée. Ce module de correction effectue une déviation de la trajectoire de manière à ce que les coordonnées en hauteur ou altitude dans le référentiel global fixe GF soient les mêmes au début et à la fin d'une foulée, et les coordonnées perpendiculaires ou dans le plan du sol également.

Le système peut, en outre, comprendre un deuxième ensemble capteur EC2 semblable au premier ensemble capteur EC1, et un deuxième module de fixation MF2 adaptés pour fixer le deuxième ensemble capteur EC2 à l'autre pied de l'utilisateur. Un module de synchronisation SYNC permet d'observer les évènements provenant des ensembles EC1 et EC2 avec une base de temps commune. On peut ainsi, à tout instant, connaître l'écart temporel entre un événement mesuré par le premier ensemble capteur EC1 (l'instant où le pied gauche va quitter le sol, par exemple) et un autre événement mesuré par le deuxième ensemble capteur EC2 (la pose du pied droit au sol, par exemple).

En outre, le système peut comprendre un troisième ensemble capteur EC3 similaire au premier ensemble capteur EC1, et un troisième module de fixation MF3 adapté pour fixer le troisième ensemble capteur EC3 à mi-hauteur de l'utilisateur, par exemple au niveau de l'abdomen.

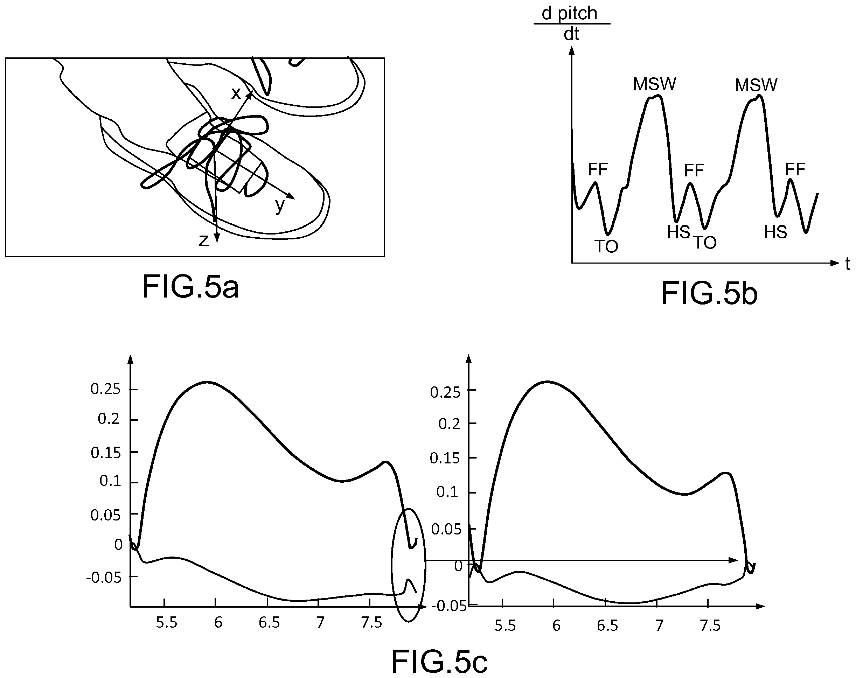

La figure 5a représente un pied muni d'un premier ensemble capteur EC1. La vitesse angulaire, ou variation temporelle de l'angle de tangage Pitch du premier ensemble capteur EC1, à l'instant t, est donnée par ($Pitch(t_{n+1})$-$Pitch(tn)$), avec $Pitch(t)$=arctan($m_{AP}/m_{VT}$), où $m_{AP}$ et $m_{VT}$ sont les mesures du magnétomètre 3M enregistrées dans le plan principal $P_f$ de la foulée (directions y et z).

La figure 5b représente la détection, par le premier module de détection DET1, des instants caractéristiques HS, FF, TO, MSW de la foulée. En début de pas FF, l'accélération est supposée nulle. Les différents instants caractéristiques cités précédemment HS, FF, TO, et MSW de la foulée sont détectés aux extrema locaux. Ce type de détection est proche de c la méthode décrite dans la demande de brevet FR 2926971.

La décomposition en valeurs singulières SVD permet de déterminer les axes principaux du mouvement. Les étapes de calcul sont :

- une étape d'autocorrélation des trois composantes du magnétomètre triaxial 3M pour obtenir la matrice de covariance, à trois lignes et trois colonnes, des signaux du magnétomètre 3M ;
- une étape de décomposition en éléments propres de la matrice de covariance, chaque valeur propre mesurant la part de variance selon le vecteur propre associé ; et
- une étape d'analyse en composantes principales, dans laquelle les données sont projetées sur chacun des axes définis par les vecteurs propres.

On détermine le plan $[\vec{u}_1,\vec{u}_2]$ de la trajectoire à partir des vecteurs délivrés par le magnétomètre triaxial 3M. Le plan $[\vec{u}_1,\vec{u}_2]$ correspond au plan contenant au maximum les mesures, considérées comme des points ou vecteurs dans un espace à trois dimensions en considérant les mesure transmises pour chaque axe du magnétomètre triaxial, et l'axe porté par le vecteur $\vec{u}_3$ est l'axe sur lequel la projection des mesures varie le moins. Le vecteur $\vec{u}_3$ est le vecteur normal au plan de la trajectoire $[\vec{u}_1,\vec{u}_2]$, mais aussi l'axe de rotation du premier ensemble capteur EC1.

Pour déterminer ces trois vecteurs $\vec{u}_1,\vec{u}_2,\vec{u}_3$, on forme une matrice $A^{LF}$ à n+1 lignes, n+1 étant le nombre d'échantillons pris entre $t_0$ et $t_n$ compris, et à 3 colonnes correspondant aux trois mesures des trois axes x, y, z du premier ensemble capteur EC1. On a :

$$A^{LF}\left(t_0,...,t_n\right) = \begin{bmatrix} A_x^{LF}(t_0) & A_y^{LF}(t_0) & A_z^{LF}(t_0) \\ \cdots & \cdots & \cdots \\ A_x^{LF}(t_n) & A_y^{LF}(t_n) & A_z^{LF}(t_n) \end{bmatrix}$$

Les mesures permettant de former la matrice $A^{LF}$ peuvent être les mesures du magnétomètre triaxial 3M ou de l'accéléromètre triaxial 3A.

En, variante, on peut ne prendre qu'un sous-ensemble des échantillons aux instants successifs $t_0$, $t_1$, ...$t_n$.

Les trois vecteurs $\vec{u}_1, \vec{u}_2, \vec{u}_3$, sont déterminés à partir de la matrice $A^{LF} (=A^{LF}(t_0,...,t_n))$, par décomposition en valeurs singulières SVD de la matrice $A^{LF}$ : $A^{LF}$ =USV. V est une matrice à trois lignes et trois colonnes dont chaque colonne correspond à un des trois vecteurs $\vec{u}_1, \vec{u}_2, \vec{u}_3$. La matrice S contient les trois valeurs singulières $s_1$, $s_2$, $s_3$. Ces trois valeurs singulières $s_1$, $s_2$, $s_3$, doivent êtres classées par ordre décroissant. Le vecteur $\vec{u}_3$ est la colonne de la matrice V correspondant à la plus petite valeur singulière. Les deux autres colonnes donnent les coordonnées des vecteurs formant le plan $P_f$ principal de la foulée [$\vec{u}_1, \vec{u}_2$].

Le troisième module de calcul CALC3 soustrait la gravité après avoir projeté l'accélération mesurée dans le référentiel fixe GF, pour extraire l'accélération propre.

A ces fins, on centre les données. En effet, on suppose avoir une vitesse nulle à chaque pied à plat FF, ie au début et à la fin de chaque foulée.

$$v(t) = \int_0^t a(u)\, du + v(0)$$

$$v(T) = \int_0^t a(u)\, du + v(0) = 0 .$$

Or                                                                           donc si T est la durée de la foulée,                                                                           Puisque

$$\sum_{i=0}^{N} a(t_i) = 0$$

$v(0) = v(T) = 0$, et en discrétisant la somme intégrale, on obtient que :                                    $\Leftrightarrow \bar{a} = 0$.

La moyenne globale de l'accélération est donc nulle sur une foulée, et il suffit donc de soustraire sa moyenne à chacune des composantes projetées sur les 3 axes du référentiel fixe, la contribution de la gravité y étant constante.

Le quatrième module de calcul CALC4 effectue une double intégration temporelle du vecteur centré des accélérations dans le référentiel global GF délivré par le troisième module de calcul CALC3, de manière à obtenir la trajectoire du premier ensemble capteur EC1 dans le référentiel global GF, sur la foulée.

La méthode d'intégration utilisée est une méthode de Simpson adaptée ou méthodes des rectangles. La première intégration permet de passer de l'accélération à la vitesse échantillon par échantillon. La deuxième intégration donne la distance à partir de la vitesse, dans les 3 dimensions.

Or cette vitesse est supposée nulle à l'instant t=0 (FF), ce qui n'est jamais le cas en pratique. Il faut rajouter une vitesse tangentielle $R.\dfrac{d\,Pitch_{R_f}}{dt}$ , R étant la distance entre le capteur et le métatarse du pied (où la vitesse est réellement nulle au posé au sol) et $\dfrac{d\,Pitch_{R_f}}{dt} =$ ($Pitch_{R_f}(t_{n+1})$-$Pitch_{R_f}(t_n)$), la vitesse de tangage $\dfrac{d\,Pitch_{R_f}}{dt}$ étant celle fournie par le troisième module de détermination DET3.

Des données expérimentales ont permis de faire une estimation de R ainsi que de l'orientation de cette vitesse dans le référentiel du premier ensemble capteur, afin de donner des contributions suffisamment précises à chacun des trois axes d'intégration.

Le module de correction CORR des dérives, après intégration, trois trajectoires sont obtenues de façon indépendante, selon les trois axes du référentiel fixe. Soit x l'axe horizontal dans la direction de la foulée, Z l'axe vertical et y l'axe horizontal perpendiculaire au plan de foulée. On fait l'hypothèse que le pied a la même orientation au début et à la fin de la foulée, et donc on redresse d'une part la trajectoire z = f(x), d'autre part y = f(x), en établissant y(T) = y(0) et z(T) = z(0).

Par exemple, comme illustré sur la figure 5c, sont représentées les courbes z = f(x), et y = f(x) : cette dernière, projetée dans le plan de la figure, est redressée pour que le dernier point soit commun aux deux trajectoires. En d'autres termes, il s'agit de la trajectoire reconstruite dans un plan perpendiculaire au plan donné par les deux directions principales de la trajectoire [$\vec{u}_1, \vec{u}_2$], avec ici une représentation simple en deux dimensions, qui est redressée, i.e. dont les coordonnées du posé de pied à la fin de la foulée sont supposées être les mêmes que celles au début de la foulée, verticalement et transversalement (le pied doit retoucher le sol et on suppose que le coureur court selon un segment de droite pour faciliter la représentation finale de la trajectoire.

Le troisième module de détermination DET3 des angles de tangage $Pitch_{R_f}$ de roulis $Roll_{R_f}$ et de lacet $Yaw_{R_f}$ du premier ensemble capteur EC1, à partir de ladite projection orthogonale PO des vecteurs délivrés par le magnétomètre triaxial 3M dans le référentiel $R_f$ lié au premier ensemble capteur EC1 pour la foulée, et de détermination des vitesses angulaires correspondantes de tangage $\dfrac{d\,Pitch_{R_f}}{dt}$, de roulis $\dfrac{d\,Roll_{R_f}}{dt}$ et de lacet $\dfrac{d\,Yaw_{R_f}}{dt}$.

Les mesures du magnétomètre triaxial 3M, projetées sur le référentiel de la foulée Rf, sont utilisées pour calculer les trois angles du mouvement :

- le tangage $Pitch_{R_f}$ : rotation autour de l'axe perpendiculaire au mouvement obtenu par arctan $(B_{U2}/B_{U1})$ ;
- le roulis $Roll_{R_f}$ : rotation autour de l'axe du sens du mouvement obtenu par arctan $(B_{U1}/B_{U3})$ ; et
- le lacet $Yaw_{R_f}$ : rotation autour de l'axe vertical obtenu par arctan $(B_{U3}/B_{U2})$ ;

avec $B_{U1}$ / $B_{U2}$ / $B_{U3}$ les composantes du champ magnétique sur les trois axes du référentiel de la foulée Rf : mU1 / mU2 / mU3.

En variante, ces angles peuvent être obtenus différemment, par exemple en obtenant la matrice de rotation mettant en jeu les trois angles, à partir des projections $B_{U1}$, $B_{U2}$ et $B_{U3}$.

Connaissant ces trois angles, on peut obtenir les vitesses angulaires $\dfrac{d\,Pitch_{R_f}}{dt}$, $\dfrac{d\,Roll_{R_f}}{dt}$ et $\dfrac{d\,Yaw_{R_f}}{dt}$ de manière instantanée.

Ces valeurs sont généralement faibles si les angles ne varient pas de façon significative. Par exemple, au cours de la phase de cycle avant, seule la variation du tangage Pitch n'est pas négligeable. Le nombre de degrés de liberté dans le mouvement y est donc inférieur ou égal à quatre. La trajectoire reconstruite peut être validée lors de cette phase.

Par contre, si deux ou trois de ces valeurs sont supérieures en valeur absolue au seuil S, et que les accélérations selon les trois dimensions sont de plus importantes, on a plus de quatre degrés de liberté, et la validité de la trajectoire est réduite.

Dans ce cas, on peut corriger la trajectoire reconstruite en affinant l'orientation de la foulée grâce à une SVD effectuée dans cette phase, qui nous donnera le plan du mouvement, différent du plan obtenu sur l'ensemble de la foulée.

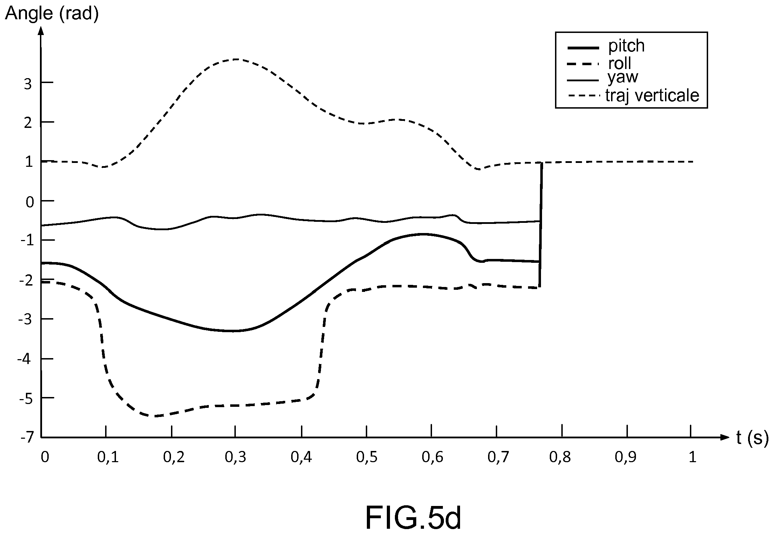

La figure 5d représente les valeurs des trois angles de tangage $Pitch_{R_f}$ de roulis $Roll_{R_f}$ et de lacet $Yaw_{R_f}$ exprimés en radians, en fonction du temps, exprimé en secondes, au cours d'une foulée, ainsi que la trajectoire verticale de cette foulée afin de visualiser les évolutions de ces angles en fonction des phases de la foulée.

Un tel système permet d'obtenir des informations, en trois dimensions, relatives à la foulée d'un utilisateur, notamment d'un coureur.

**Revendications**

1. Système d'analyse de foulées d'un utilisateur, comprenant :

   - un premier ensemble capteur (EC1) muni d'un boîtier, d'un magnétomètre triaxial (3M) lié fixement audit boîtier (BT), et d'un référentiel mobile (LF) ; et
   - des premiers moyens de fixation (MF1) adaptés pour fixer le premier ensemble capteur (EC1) à un segment de jambe de l'utilisateur ;
   - des moyens de traitement (MT) adaptés pour calculer l'angle de lacet ($Yaw_{GF}$) et/ou de tangage ($Pitch_{GF}$) et/ou de roulis ($Roll_{GF}$) dudit premier ensemble capteur (EC1), et pour calculer la ou les variations temporelles angulaires correspondantes (

   $\dfrac{d\,Yaw_{GF}}{dt}$, $\dfrac{d\,Pitch_{GF}}{dt}$, $\dfrac{d\,Roll_{GF}}{dt}$

   ), dans un référentiel global fixe (GF) lié au référentiel terrestre, à partir des mesures dudit magnétomètre triaxial (3M) délivrées dans ledit référentiel mobile (LF) ;

   dans lequel :

   lesdits moyens de traitement (MT) comprennent :

   - des premiers moyens de calcul (CALC1) de la variation temporelle de l'angle de tangage (Pitch) du premier ensemble capteur (EC1) à partir des mesures dudit magnétomètre triaxial (3M) délivrées dans ledit référentiel mobile (LF); et
   - des premiers moyens de détermination (DET1) configurés pour déterminer un instant de début d'une foulée et d'un instant de fin de ladite foulée et de phases de ladite foulée, à partir de ladite variation temporelle de l'angle de tangage (Pitch) du premier ensemble capteur (EC1) fournie par lesdits premiers moyens de calcul;

ledit premier ensemble capteur (EC1) est, en outre, muni d'un accéléromètre triaxial (3A) lié fixement audit boîtier (BT) ; et

lesdits moyens de traitement (MT) comprennent des deuxièmes moyens de détermination (DET2) configurés pour déterminer des valeurs de paramètres représentatifs d'une phase de ladite foulée ou de la foulée complète, en trois dimensions, dans le référentiel global fixe (GF);

une foulée comprenant une phase d'appui du pied auquel est fixé le premier ensemble capteur (EC1), suivi d'une phase de cycle arrière dans laquelle ledit pied remonte du sol en direction du dos de l'utilisateur, et d'une phase de cycle avant dans laquelle ledit pied se dirige devant l'utilisateur, lesdits deuxièmes moyens de détermination (DET2) comprenant des premiers moyens d'estimation (EST1) d'un référentiel lié au premier ensemble capteur (EC1), pour la foulée ($R_f$) et/ou par phase ($R_{ap}$, $R_{ar}$, $R_{av}$) de la foulée construit à partir de deux axes du plan principal ($P_t$) de la foulée et d'un axe normal, à partir d'une décomposition en valeurs singulières (SVD), de la matrice à trois colonnes formée par des vecteurs délivrés par ledit magnétomètre triaxial (3M) à des instants successifs, dans le référentiel mobile (LF) lié au premier ensemble capteur (EC1) ; lesdits moyens de traitement (MT) comprenant des deuxièmes moyens de calcul (CALC2) de projections orthogonales (PO) des vecteurs de mesures délivrés par l'accéléromètre triaxial (3A) et par le magnétomètre triaxial (3M) sur les axes du référentiel lié au premier ensemble capteur sur la foulée ($R_f$) ; et

le référentiel global fixe (GF) lié au référentiel terrestre étant ladite projection orthogonale (PO) des vecteurs délivrés par le magnétomètre triaxial (3M) dans le référentiel lié au premier ensemble capteur (EC1) pour la foulée ($R_f$).

2. Système selon la revendication 1, dans lequel lesdits deuxièmes moyens de détermination (DET2) comprennent des troisièmes moyens de détermination (DET3) des angles de tangage ($Pitch_{R_f}$), de roulis ($Roll_{R_f}$) et de lacet ($Yaw_{R_f}$) du premier ensemble capteur (EC1), à partir de ladite projection orthogonale (PO) des vecteurs délivrés par le magnétomètre triaxial (3M) dans le référentiel ($R_f$) lié au premier ensemble capteur (EC1) pour la foulée , et de détermination des vitesses angulaires correspondantes de tangage $\left( \dfrac{d\,Pitch_{R_f}}{dt} \right)$, de roulis $\left( \dfrac{d\,Roll_{R_f}}{dt} \right)$ et de lacet $\left( \dfrac{d\,Yaw_{R_f}}{dt} \right)$.

3. Système selon la revendication 2, dans lequel lesdits deuxièmes moyens de détermination (DET2) comprennent des moyens de comparaison (COMP) desdites vitesses angulaires de tangage $\left( \dfrac{d\,Pitch_{R_f}}{dt} \right)$, de roulis

$\left( \dfrac{d\,Roll_{R_f}}{dt} \right)$ et de lacet $\left( \dfrac{d\,Yaw_{R_f}}{dt} \right)$ avec un seuil (S).

4. Système selon la revendication 3, dans lequel, lesdits deuxièmes moyens de détermination (DET2) comprennent des moyens d'utilisation (UTIL) du référentiel ($R_{ap}$, $R_{ar}$, $R_{av}$) lié au premier ensemble capteur (EC1) pour la phase en cours, lorsqu'au moins deux desdites valeurs absolues des trois vitesses angulaires sont supérieures audit seuil (S), pour redresser l'orientation par compensation des déviations de la trajectoire, dues aux rotations, par rapport au référentiel global (GF) en utilisant ledit référentiel ($R_{ap}$, $R_{ar}$, $R_{av}$) lié audit premier ensemble capteur (EC1) pour la phase en cours à la place du référentiel ($R_f$) lié au premier ensemble capteur (EC1) pour la foulée.

5. Système selon l'une des revendications précédentes, dans lequel lesdits premiers moyens de détermination (DET1) sont adaptés pour identifier des instants caractéristiques (HS, FF, TO, MSW) de la foulée de l'utilisateur par détection d'extrema locaux de la variation temporelle $\left(\dfrac{d\ pitch}{dt}\right)$ de l'angle de tangage (Pitch) du premier ensemble capteur (EC1).

6. Système selon l'une des revendications précédentes, dans lequel les deuxièmes moyens de détermination (DET2) comprennent des troisièmes moyens de calcul (CALC3) pour soustraire, au vecteur correspondant à la projection orthogonale (PO) dans le référentiel global (GF) du vecteur de mesures de l'accéléromètre triaxial (3A) délivré par les deuxièmes moyens de calcul (CALC2), le vecteur moyen sur la foulée de la projection orthogonale (PO) dans le référentiel global (GF) du vecteur de mesures de l'accéléromètre triaxial (3A) délivré par les deuxièmes moyens de calcul (CALC2), de manière à obtenir le vecteur centré des accélérations dans le référentiel global (GF).

7. Système selon l'une des revendications précédentes, dans lequel les deuxièmes moyens de détermination (DET2) comprennent des quatrièmes moyens de calcul (CALC4) d'une double intégration temporelle dudit vecteur centré des accélérations dans le référentiel global (GF) délivré par lesdits troisièmes moyens de calcul (CALC3), de manière à obtenir la trajectoire du premier ensemble capteur (EC1) dans ledit référentiel global (GF), sur la foulée.

8. Système selon l'une des revendications précédentes dans lequel les deuxièmes moyens de détermination (DET2) comprennent des moyens de correction (CORR) des dérives du magnétomètre (3M) et de l'accéléromètre (3A), en supposant que le pied de l'utilisateur a une orientation identique en début et fin de foulée, par redressement, de manière à avoir des coordonnées verticales et transversales identiques en début et fin de foulée.

9. Système selon l'une des revendications précédentes, comprenant un deuxième ensemble capteur (EC2) semblable au premier ensemble capteur (EC1), et des deuxièmes moyens de fixation (MF2) adaptés pour fixer le deuxième ensemble capteur (EC2) à un segment de l'autre jambe de l'utilisateur.

10. Système selon la revendication 9, comprenant, en outre des moyens de synchronisation (SYNC) pour comparer les mesures effectuées par le ou lesdits ensembles capteurs d'une jambe par rapport à l'autre.

11. Système selon la revendication 9 ou 10, comprenant, en outre un troisième ensemble capteur (EC3) similaire au premier ensemble capteur, et des troisièmes moyens de fixation (MF3) adaptés pour fixer le troisième ensemble capteur (EC3) à mi-hauteur de l'utilisateur.

12. Procédé d'analyse de foulées d'un utilisateur, dans lequel :

- on calcule l'angle de lacet (YawGF) et/ou de tangage (PitchGF) et/ou de roulis (RollGF) ainsi que la ou les variations temporelles angulaires correspondantes $\left(\dfrac{d\ Yaw_{GF}}{dt}, \dfrac{d\ Pitch_{GF}}{dt}, \dfrac{d\ Roll_{GF}}{dt}\right),$ dans un référentiel global fixe (GF) lié au référentiel terrestre, d'un premier ensemble capteur (EC1), comprenant un magnétomètre triaxial (3M) et un accéléromètre triaxial (3A), fixé à un segment de jambe de l'utilisateur, à partir des mesures dudit magnétomètre triaxial (3M) ;
- on détermine un instant de début d'une foulée et un instant de fin de ladite foulée et des phases de ladite foulée, à partir de calcul de la variation temporelle $\left(\dfrac{d\ pitch}{dt}\right)$ de l'angle de tangage (Pitch) du premier ensemble capteur (EC1) ;
- on calcule (CALC1) la variation temporelle de l'angle de tangage (Pitch) du premier ensemble capteur (EC1) à partir des mesures dudit magnétomètre triaxial (3M) délivrées dans ledit référentiel mobile (LF);
- on détermine (DET1) un instant de début d'une foulée et un instant de fin de ladite foulée et de phases de ladite foulée, à partir de ladite variation temporelle de l'angle de tangage (Pitch) du premier ensemble capteur (EC1) fournie par lesdits premiers moyens de calcul;
- on détermine (DET2) des valeurs de paramètres représentatifs d'une phase de ladite foulée ou de la foulée complète, en trois dimensions, dans le référentiel global fixe (GF);

une foulée comprenant une phase d'appui du pied auquel est fixé le premier ensemble capteur (EC1), suivi d'une phase de cycle arrière dans laquelle ledit pied remonte du sol en direction du dos de l'utilisateur, et d'une phase de cycle avant dans laquelle ledit pied se dirige devant l'utilisateur, ladite détermination (DET2) des valeurs de paramètres comprend une estimation (EST1) d'un référentiel lié au premier ensemble capteur (EC1), pour la foulée ($R_f$) et/ou par phase ($R_{ap}$, $R_{ar}$, $R_{av}$) de la foulée, à partir d'une décomposition en valeurs singulières (SVD), de la matrice à trois colonnes formée par des vecteurs délivrés par ledit magnétomètre triaxial (3M) à des instants successifs, dans le référentiel mobile (LF) lié au premier ensemble capteur (EC1).

**Patentansprüche**

1. System zur Analyse der Schritte eines Benutzers, Folgendes beinhaltend:

   - eine erste Sensorbaugruppe (EC1), versehen mit einem Gehäuse, einem Triaxial-Magnetometer (3M), welches mit dem Gehäuse (BT) fest verbunden ist, und einem mobilen Bezugssystem (LF); und
   - erste Befestigungsmittel (MF1), welche geeignet sind, die erste Sensorbaugruppe (EC1) an einem Beinsegment des Benutzers zu befestigen;
   - Bearbeitungsmittel (MT), welche geeignet sind, den Gierwinkel ($Yaw_{GF}$) und/oder den Nickwinkel ($Pitch_{GF}$) und/oder den Rollwinkel ($Roll_{GF}$) der ersten Sensorbaugruppe (EC1) zu berechnen, und zum Berechnen der entsprechenden zeitlichen Winkel-Variation bzw. Winkel-Variationen $\left( \dfrac{d\,Yaw_{GF}}{dt}, \dfrac{d\,Pitch_{GF}}{dt}, \dfrac{d\,\overline{Roll}_{GF}}{dt} \right)$, in einem festen globalen Bezugssystem (GF), welches mit dem Erd-Bezugssystem verbunden ist, anhand der Messungen des Triaxial-Magnetometers (3M), welche im mobilen Bezugssystem (LF) abgegeben werden;

   bei welchem:

   die Bearbeitungsmittel (MT) Folgendes beinhalten:

   - erste Berechnungsmittel (CALC1) der zeitlichen Variation des Nickwinkels (Pitch) der ersten Sensorbaugruppe (EC1) anhand der Messungen des Triaxial-Magnetometers (3M), welche im mobilen Bezugssystem (LF) abgegeben werden; und
   - erste Bestimmungsmittel (DET1), konfiguriert zum Bestimmen eines Anfangsmomentes eines Schrittes und eines Endmomentes des Schrittes und von Phasen des Schrittes anhand der zeitlichen Variation des Nickwinkels (Pitch) der ersten Sensorbaugruppe (EC1), welche durch die ersten Berechnungsmittel bereitgestellt wird;

   wobei die erste Sensorbaugruppe (EC1) zudem mit einem Triaxial-Beschleunigungsmesser (3A) versehen ist, welches fest mit dem Gehäuse (BT) verbunden ist; und
   die Bearbeitungsmittel (MT) zweite Bestimmungsmittel (DET2) beinhalten, konfiguriert zum Bestimmen der Werte von Parametern, welche eine Phase des Schrittes oder den kompletten Schritt in drei Dimensionen in dem festen Bezugssystem (GF) darstellen;
   wobei ein Schritt eine Phase des Aufstützens des Fußes beinhaltet, an welchem die erste Sensorbaugruppe (EC1) befestigt ist, gefolgt von einer Zyklusphase nach hinten, bei welcher der Fuß vom Boden in Richtung des Rückens des Benutzers wieder ansteigt, und einer Zyklusphase nach vorn, bei welcher der Fuß sich vor den Benutzer richtet, wobei die zweiten Bestimmungsmittel (DET2) erste Schätzmittel (EST1) eines mit einer ersten Sensorbaugruppe (EC1) verbundenen Bezugssystems beinhalten, für den Schritt ($R_f$) und/oder pro Phase ($R_{ap}$, $R_{ar}$, $R_{av}$) des Schrittes, konstruiert anhand von zwei Achsen der Hauptebene ($P_t$) des Schrittes und einer normalen Achse, anhand einer Zerlegung in singuläre Werte (SVD), der dreispaltigen Matrix, welche durch Vektoren gebildet wird, die durch das Triaxial-Magnetometer (3M) zu aufeinander folgenden Zeitpunkten in dem mobilen Bezugssystem (LF), welches mit der ersten Sensorbaugruppe (EC1) verbunden ist, abgegeben werden; wobei die Bearbeitungsmittel (MT) zweite Berechnungsmittel (CALC2) von rechtwinkligen Projektionen (PO) der vom Triaxial-Beschleunigungsmesser (3A) und vom Triaxial-Magnetometer (3M) auf den Achsen des mit der ersten Sensorbaugruppe verbundenen Bezugssystems am Schritt ($R_f$) abgegebenen Messvektoren beinhaltet; und
   wobei das globale feste Bezugssystem (GF), welches mit dem Erd-Bezugssystem verbunden ist, die rechtwinklige Projektion (PO) der vom Triaxial-Magnetometer (3M) in dem mit der ersten Sensorbaugruppe (EC1) verbundenen Bezugssystem abgegebenen Vektoren für den Schritt ($R_f$) ist.

**2.** System nach Anspruch 1, bei welchem die zweiten Bestimmungsmittel (DET2) dritte Bestimmungsmittel (DET3) zur Bestimmung des Nickwinkels (*Pitch$_{Rf}$*), des Rollwinkels (*Roll$_{Rf}$*) und des Gierwinkels (*Yaw$_{Rf}$*) der ersten Sensorbaugruppe (EC1) beinhalten, anhand der rechtwinkligen Projektion (PO) der vom Triaxial-Magnetometer (3M) in dem mit der ersten Sensorbaugruppe (EC1) verbundenen Bezugssystem (R$_f$) für den Schritt abgegebenen Vektoren, und zur Bestimmung der entsprechenden Nick- $\left(\dfrac{d\,Pitch_{R_f}}{dt}\right)$, Roll- $\left(\dfrac{d\,Roll_{R_f}}{dt}\right)$ und Gier-Winkelgeschwindigkeiten $\left(\dfrac{d\,Yaw_{R_f}}{dt}\right)$.

**3.** System nach Anspruch 2, bei welchem die zweiten Bestimmungsmittel (DET2) Vergleichsmittel (COMP) der Nick- $\left(\dfrac{d\,Pitch_{R_f}}{dt}\right)$, Roll- $\left(\dfrac{d\,Roll_{R_f}}{dt}\right)$ und Gier-Winkelgeschwindigkeiten $\left(\dfrac{d\,Yaw_{R_f}}{dt}\right)$ mit einem Schwellenwert (S) besitzen.

**4.** System nach Anspruch 3, bei welchem die zweiten Bestimmungsmittel (DET2) Benutzungsmittel (UTIL) des Bezugssystems (R$_{ap}$, R$_{ar}$, R$_{av}$), welches mit der ersten Sensorbaugruppe (EC1) für die augenblickliche Phase verbunden ist, beinhalten, wenn mindestens zwei der Absolutwerte der drei Winkelgeschwindigkeiten den Schwellenwert (S) überschreiten, um die Ausrichtung zum globalen Bezugssystem (GF) durch Ausgleich der Bahnabweichungen aufgrund von Drehungen zu berichtigen, indem das Bezugssystem (R$_{ap}$, R$_{ar}$, R$_{av}$), welches mit der ersten Sensorbaugruppe (EC1) für die augenblickliche Phase verbunden ist, anstelle des mit der ersten Sensorbaugruppe (EC1) verbundenen Bezugssystems (R$_f$) für den Schritt benutzt wird.

**5.** System nach einem der vorhergehenden Ansprüche, bei welchem die ersten Bestimmungsmittel (DET1) geeignet sind, um charakteristische Momente (HS, FF, TO, MSW) des Schrittes des Benutzers durch Erkennung lokaler Extremwerte in der zeitlichen Variation $\left(\dfrac{d\,pitch}{dt}\right)$ des Nickwinkels (Pitch) der ersten Sensorbaugruppe (EC1) zu identifizieren.

**6.** System nach einem der vorhergehenden Ansprüche, bei welchem die zweiten Bestimmungsmittel (DET2) dritte Berechnungsmittel (CALC3) beinhalten zur Subtraktion, von dem der rechtwinkligen Projektion (PO) im globalen Bezugssystem (GF) entsprechenden Vektor des Messvektors des Triaxial-Beschleunigungsmessers (3A), welcher durch die zweiten Berechnungsmittel (CALC2) abgegeben wird, des mittleren Vektors über den Schritt der rechtwinkligen Projektion (PO) im globalen Bezugssystem (GF) des Messvektors des Triaxial-Beschleunigungsmessers (3A), welcher durch die zweiten Berechnungsmittel (CALC2) abgegeben wird, in der Weise, dass der zentrierte Vektor der Beschleunigungen im globalen Bezugssystem (GF) erzielt wird.

**7.** System nach einem der vorhergehenden Ansprüche, bei welchem die zweiten Bestimmungsmittel (DET2) vierte Berechnungsmittel (CALC4) beinhalten zur Berechnung einer doppelten zeitlichen Integration des zentrierten Vektors der Beschleunigungen im globalen Bezugssystem (GF), welcher durch die dritten Berechnungsmittel (CALC3) abgegeben wird, in der Weise, dass die Bahn der ersten Sensorbaugruppe (EC1) im globalen Bezugssystem (GF) über den Schritt erzielt wird.

**8.** System nach einem der vorhergehenden Ansprüche, bei welchem die zweiten Bestimmungsmittel (DET2) Korrekturmittel (CORR) der Drifts des Magnetometers (3M) und des Beschleunigungsmessers (3A) durch Berichtigung beinhalten, in der Annahme, dass der Fuß des Benutzers eine gleiche Ausrichtung zu Anfang und Ende des Schrittes besitzt, in der Weise, dass man zu Anfang und zu Ende des Schrittes identische vertikale und transversale Koordinaten erhält.

9. System nach einem der vorhergehenden Ansprüche, beinhaltend eine zweite Sensorbaugruppe (EC2), welche der ersten Sensorbaugruppe (EC1) ähnlich ist, und zweite Befestigungsmittel (MF2), welche geeignet sind, die zweite Sensorbaugruppe (EC2) an einem Segment des anderen Beins des Benutzers zu befestigen.

10. System nach Anspruch 9, zudem beinhaltend Synchronisierungsmittel (SYNC) zum Vergleichen der durch die Sensorbaugruppe(n) eines Beins mit der- bzw. denjenigen des anderen Beins vorgenommenen Messungen.

11. System nach Anspruch 9 oder 10, zudem beinhaltend eine dritte Sensorbaugruppe (EC3), welche der ersten Sensorbaugruppe ähnlich ist, und dritte Befestigungsmittel (MF3), welche geeignet sind, die dritte Sensorbaugruppe (EC3) in halber Höhe des Benutzers zu befestigen.

12. Verfahren zur Analyse der Schritte eines Benutzers, bei welchem:

- man den Gierwinkel ($Yaw_{GF}$) und/oder den Nickwinkel ($Pitch_{GF}$) und/oder den Rollwinkel ($Roll_{GF}$) sowie die entsprechende(n) zeitliche(n) Winkel-Variation(en)

$$\left( \frac{d\,Yaw_{GF}}{dt}, \frac{d\,Pitch_{GF}}{dt}, \frac{d\,Roll_{GF}}{dt} \right)$$

berechnet, in einem festen, globalen Bezugssystem (GF), welches mit dem Erd-Bezugssystem verbunden ist, einer ersten Sensorbaugruppe (EC1), welche ein Triaxial-Magnetometer (3M) und einen Triaxial-Beschleunigungsmesser (3A) beinhaltet, welche an einem Beinsegment des Benutzers befestigt ist, anhand von Messungen des Triaxial-Magnetometers (3M);
- man einen Anfangsmoment eines Schrittes und einen Endmoment des Schrittes und von Phasen des Schrittes anhand der Berechnung der zeitlichen Variation $\left( \frac{d\,pitch}{dt} \right)$ des Nickwinkels (Pitch) der ersten Sensorbaugruppe (EC1) bestimmt;
- man die zeitliche Variation des Nickwinkels (Pitch) der ersten Sensorbaugruppe (EC1) anhand der Messungen des Triaxial-Magnetometers (3M) berechnet (CALC1), welche im mobilen Bezugssystem (LF) abgegeben werden;
- man einen Anfangsmoment eines Schrittes und einen Endmoment des Schrittes und von Phasen des Schrittes anhand der zeitlichen Variation des Nickwinkels (Pitch) der ersten Sensorbaugruppe (EC1) bestimmt (DET1), welche durch die ersten Berechnungsmittel bereitgestellt wird;
- man Werte von Parametern bestimmt (DET2), welche eine Phase des Schrittes oder den kompletten Schritt in drei Dimensionen in dem festen Bezugssystem (GF) darstellen;

wobei ein Schritt eine Phase des Aufstützens des Fußes beinhaltet, an welchem die erste Sensorbaugruppe (EC1) befestigt ist, gefolgt von einer Zyklusphase nach hinten, bei welcher der Fuß sich vom Boden in Richtung des Rückens des Benutzers wieder hebt, und einer Zyklusphase nach vorn, bei welcher der Fuß vor den Benutzer geführt wird, wobei die Bestimmung (DET2) der Parameterwerte eine Schätzung (EST1) eines Bezugssystems beinhaltet, welches mit der ersten Sensorbaugruppe (EC1) verbunden ist, für den Schritt ($R_f$) und/oder pro Phase ($R_{ap}$, $R_{ar}$, $R_{av}$) des Schrittes, anhand einer Zerlegung in singuläre Werte (SVD) der dreispaltigen Matrix, welche durch Vektoren gebildet wird, die vom Triaxial-Magnetometer (3M) zu aufeinander folgenden Zeitpunkten in dem mit der ersten Sensorbaugruppe (EC1) verbundenen mobilen Bezugssystem (LF) abgegeben werden.

**Claims**

1. A system for analysing the strides of a user, comprising:

- a first sensor assembly (EC1) provided with a housing, a triaxial magnetometer (3M) fixedly connected to said housing (BT) and a movable frame (LF); and
- first attachment means (MF1) adapted to attach the first sensor assembly (EC1) to a section of a leg of the user; and
- processing means (MT) adapted to compute the yaw ($Yaw_{GF}$) and/or pitch ($Pitch_{GF}$) and/or roll ($Roll_{GF}$) angle of said first sensor assembly (EC1) and to compute the one or more corresponding angular temporal variation(s)

$$\left(\frac{d\,Yaw_{GF}}{dt}, \frac{d\,Pitch_{GF}}{dt}, \frac{d\,Roll_{GF}}{dt}\right)$$ in a fixed global frame (GF) connected to the terrestrial frame, on the basis of measurements of said triaxial magnetometer (3M) delivered in said movable frame (LF);

wherein:

said processing means (MT) comprise:

- first computing means (CALC1) for computing the temporal variation of the pitch angle (Pitch) of the first sensor assembly (EC1), on the basis of measurements of said triaxial magnetometer (3M) delivered in said movable frame (LF); and
- first determining means (DET1) configured to determine a start instant of a stride and an end instant of said stride and phases of said stride, on the basis of said temporal variation of the pitch angle (Pitch) of the first sensor assembly (EC1) supplied by said first computation means;

said first sensor assembly (EC1) being further provided with a triaxial accelerometer (3A) fixedly connected to said housing (BT); and
said processing means (MT) comprising second determining means (DET2) configured to determine values of parameters representing a phase of said stride or of the full stride, in three dimensions, in the fixed global frame (GF);
a stride comprising a support phase for the foot to which the first sensor assembly (EC1) is attached, followed by a rearwards cycle phase, in which said foot leaves the ground towards the back of the user, and a forwards cycle phase, in which said foot is pointed in front of the user, said second determining means (DET2) comprising first estimating means (EST1) for estimating a frame connected to the first sensor assembly (EC1), for the stride $(R_f)$ and/or per phase $(R_{ap}, R_{ar}, R_{av})$ of the stride constructed on the basis of two axes of the main plane $(P_t)$ of the stride and a normal axis, on the basis of a breakdown, into singular values (SVD), of the three column matrix formed by vectors delivered by said triaxial magnetometer (3M) at successive instants, in the movable frame (LF) connected to the first sensor assembly (EC1), said processing means (MT) comprising second computing means (CALC2) for computing orthogonal projections (PO) of the measurement vectors delivered by the triaxial accelerometer (3A) and by the triaxial magnetometer (3M) on the axes of the frame connected to the first sensor assembly over the stride $(R_f)$; and
the fixed global frame (GF) connected to the terrestrial frame being said orthogonal projection (PO) of the vectors delivered by the triaxial magnetometer (3M) in the frame connected to the first sensor assembly (EC1) for the stride $(R_f)$.

2. The system as claimed in claim 1, wherein said second determining means (DET2) comprise third determining means (DET3) for determining pitch ($Pitch_{Rf}$), roll ($Roll_{Rf}$) and yaw ($Yaw_{Rf}$) angles of the first sensor assembly (EC1), on the basis of said orthogonal projection (PO) of the vectors delivered by the triaxial magnetometer (3M) in the frame $(R_f)$ associated with the first sensor assembly (EC1) for the stride, and for determining corresponding angular speeds of the pitch $\left(\dfrac{d\,Pitch_{R_f}}{dt}\right)$, roll $\left(\dfrac{d\,Roll_{R_f}}{dt}\right)$ and yaw $\left(\dfrac{d\,Yaw_{R_f}}{dt}\right)$.

3. The system as claimed in claim 2, wherein said second determining means (DET2) comprise comparing means (COMP) for comparing said angular speeds of the pitch $\left(\dfrac{d\,Pitch_{R_f}}{dt}\right)$, roll $\left(\dfrac{d\,Roll_{R_f}}{dt}\right)$ and yaw $\left(\dfrac{d\,Yaw_{R_f}}{dt}\right)$ to a threshold (S).

4. The system as claimed in claim 3, wherein said second determining means (DET2) comprise operating means (UTIL) for the frame $(R_{ap}, R_{ar}, R_{av})$ connected to the first sensor assembly (EC1) for the ongoing phase, when at least two of said absolute values of the three angular speeds are greater than said threshold (S), in order to rectify the orientation by compensating for drifts of the trajectory, due to the rotations, relative to the global frame (GF), by

using said frame ($R_{ap}$, $R_{ar}$, $R_{av}$) connected to said first sensor assembly (EC1) for the ongoing phase instead of the frame ($R_f$) connected to the first sensor assembly (EC1) for the stride.

5. The system as claimed in any one of the preceding claims, wherein said first determining means (DET1) are adapted to identify characteristic instants (HS, FF, TO, MSW) of the stride of the user by detecting local extrema of the temporal variation $\left( \dfrac{d\ pitch}{dt} \right)$ of the pitch angle (Pitch) of the first sensor assembly (EC1).

6. The system as claimed in any one of the preceding claims, wherein the second determining means (DET2) comprise third computing means (CALC3) for subtracting, from the vector corresponding to the orthogonal projection (PO) in the global frame (GF) of the measurements vector of the triaxial accelerometer (3A) delivered by the second computing means (CALC2), the mean vector over the stride of the orthogonal projection (PO) in the global frame (GF) of the measurements vector of the triaxial accelerometer (3A) delivered by the second computing means (CALC2), so as to obtain the centred vector of accelerations in the global frame (GF).

7. The system as claimed in any one of the preceding claims, wherein the second determining means (DET2) comprise fourth computing means (CALC4) for computing a dual temporal integration of said centred vector of accelerations in the global frame (GF) delivered by said third computing means (CALC3), so as to obtain the trajectory of the first sensor assembly (EC1) in said global frame (GF) over the stride.

8. The system as claimed in any one of the preceding claims, wherein the second determining means (DET2) comprise means (CORR) for correcting drifts of the magnetometer (3M) and of the accelerometer (3A), assuming that the foot of the user has an identical orientation at the start and at the end of the strides, by rectification, so as to have identical vertical and transverse coordinates at the start and at the end of the stride.

9. The system as claimed in any one of the preceding claims, comprising a second sensor assembly (EC2) similar to the first sensor assembly (EC2) and second attachment means (MF2) adapted to attach the second sensor assembly (EC2) to a section of the other leg of the user.

10. The system as claimed in claim 9, further comprising synchronisation means (SYNC) for comparing the measurements carried out by the sensor assembly or assemblies from one leg relative to the other.

11. The system as claimed in claim 9 or 10, further comprising a third sensor assembly (EC3) similar to the first sensor assembly and third attachment means (MF3) adapted to attach the third sensor assembly (EC3) at the mid-height of the user.

12. A method for analysing the strides of a user, wherein:

- the yaw ($Yaw_{GF}$) and/or pitch ($Pitch_{GF}$) and/or roll ($Roll_{GF}$) angle and the one or more corresponding angular temporal variation(s) $\left( \dfrac{d\ Yaw_{GF}}{dt}, \dfrac{d\ Pitch_{GF}}{dt}, \dfrac{d\ Roll_{GF}}{dt} \right)$ are computed, in a fixed global frame (GF) connected to the terrestrial frame of a first sensor assembly (EC1), comprising a triaxial magnetometer (3M) and a triaxial accelerometer (3A) attached to a section of a leg of the user, on the basis of measurements of said triaxial magnetometer (3M);
- a stride start instant and a stride end instant and phases of said stride are determined, on the basis of a computation of the temporal variation $\left( \dfrac{d\ pitch}{dt} \right)$ of the pitch angle (Pitch) of the first sensor assembly (EC1);
- the temporal variation of the pitch angle (Pitch) of the first sensor assembly (EC1) is computed (CALC1), on the basis of the measurements of said triaxial magnetometer (3M) delivered in said movable frame (LF);
- a start instant of a stride and an end instant of said stride and phases of said stride are determined (DET1), on the basis of said temporal variation of the pitch angle (Pitch) of the first sensor assembly (EC1) supplied by said first computation means;

- values of parameters representing a phase of said stride or of the full stride are determined (DET2), in three dimensions, in the fixed global frame (GF);

a stride comprising a support phase for the foot to which the first sensor assembly (EC1) is attached, followed by a rearwards cycle phase, in which said foot leaves the ground towards the back of the user, and a forwards cycle phase, in which said foot is pointed in front of the user, said determining (DET2) of the values of parameters comprising an estimate (EST1) of a frame connected to the first sensor assembly (EC1), for the stride ($R_f$) and/or per phase ($R_{ap}$, $R_{ar}$, $R_{av}$) of the stride, on the basis of a breakdown, into singular values (SVD), of the three column matrix formed by vectors delivered by said triaxial magnetometer (3M) at successive instants, in the movable frame (LF) connected to the first sensor assembly (EC1).

Pas — Pas

Foulée

## FIG.1a

← Appui droit →←— Suspension —→←— Appui gauche →

←— Cycle arrière —→←— Cycle avant →

## FIG.1b

Pose sol   A plat   Quitte sol   Séparation cycles avant/arrière

## FIG.1c

Plan sagittal

$R_f$

$P_f$

x   y   z

Trajectoire du pied

0   -0.05   -0.1   0

## FIG.2

MF1

EC1

| 3A | 3M |

MT

BT

## FIG.3a

MF1

EC1

| 3A | 3M |

BT

MT

## FIG.3b

FIG.4

FIG.5a

FIG.5b

FIG.5c

EP 2 560 551 B1

Angle (rad)

pitch
roll
yaw
traj verticale

t (s)

FIG.5d

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 0857181

- FR 2926971

**Littérature non-brevet citée dans la description**

- **A. MAALI ; A. OULDALI ; H. MIMOUN ; G. BAUDOIN.** An evaluation of UWB localization under non line-of-sight (NLOS) propagation. *Wireless Pervasive Computing, ISWPC, 3rd International Symposium on,* Mai 2008, 379-382

- **E. FOXLIN.** Inertial head-tracker sensor fusion by a complementary separate-bias kalman filter. *Virtual Reality Annual International Symposium, Proceedings of the IEEE 1996,* Mars 1996, 185-194

- **K. KOGURE ; L. SEON-WOO ; K. MASE.** Detection of spatio-temporal gait parameters by using wearable motion sensors. *Engineering in Medicine and Biology Society. IEEE-EMBS 2005. 27th Annual International Conference,* 2005, 6836-6839

- Pedestrian tracking with shoe-mounted inertial sensors. **E. FOXLIN.** Computer Graphics and Applications. IEEE, Novembre 2005, vol. 25, 38-46

- **P. ROBERTSON ; B. KRACH.** Integration of foot-mounted inertial sensors into a Bayesian location estimation framework. *Positioning, Navigation and Communication, 2008. WPNC 2008. 5th Workshop on,* Mars 2008, 55-61

- **S. SCAPELLATO ; F. CAVALLO ; A.M. SABATINI ; C. MARTELLONI.** Assessment of walking features from foot inertial sensing. *Biomedical Engineering, IEEE Transactions on,* Mars 2005, 486-494

- **D. ALVAREZ ; A.M LOPEZ ; J. RODRIGUEZ-URIA ; J.C. ALVAREZ ; R.C. GONZALEZ.** Multisensor approach to walking distance estimation with foot inertial sensing. *Engineering in Medicine and Biology Society, 2007, EMBS 2007. 29th Annual International Conference of the IEEE,* Août 2007, 5719-5722

- **K. FYFE ; GÉRARD LACHAPELLE ; R. STIRLING ; J. COLLIN.** An innovative shoe-mounted pedestrian navigation system. *Proc. European Navigation Conf. (GNSS),* Avril 2003